(19) Europäisches Patentamt
European Patent Office
Office européen des brevets

(11) **EP 2 459 284 B1**

(12) **EUROPEAN PATENT SPECIFICATION**

(45) Date of publication and mention
of the grant of the patent:
**01.07.2020 Bulletin 2020/27**

(21) Application number: **10752929.9**

(22) Date of filing: **30.07.2010**

(51) Int Cl.:
***A63B 24/00*** *(2006.01)*

(86) International application number:
**PCT/IB2010/053471**

(87) International publication number:
**WO 2011/013099 (03.02.2011 Gazette 2011/05)**

(54) **SYSTEM FOR PROVIDING A TRAINING PROGRAM TO A SUBJECT**

SYSTEM ZUR BEREITSTELLUNG EINES SCHULUNGSPROGRAMM FÜR EIN SUBJEKT

SYSTÈME POUR ATTRIBUER UN PROGRAMME D ENTRAÎNEMENT À UN SUJET

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **31.07.2009 CN 200910161110**

(43) Date of publication of application:
**06.06.2012 Bulletin 2012/23**

(73) Proprietor: **Koninklijke Philips N.V.
5656 AG Eindhoven (NL)**

(72) Inventors:
• **CHEN, Xi
Shanghai 200233 (CN)**
• **PAS, Adriana Johanna
5656 AA Eindhoven (NL)**
• **CHEN, Ningjiang
Shanghai 200233 (CN)**
• **SONG, Rong
Shanghai 200233 (CN)**

(74) Representative: **Kroeze, Johannes Antonius
Philips Intellectual Property & Standards
High Tech Campus 5
5656 AE Eindhoven (NL)**

(56) References cited:
**WO-A1-2007/140490     WO-A1-2008/129442
WO-A1-2009/127788**

**Description**

**Field of the Invention**

The present application relates to a training system, more particularly, to a system for providing a training program to a subject.

**Background of the Invention**

At present various devices are known to provide exercises for a subject, e.g. a patient who suffers from loss of motor function as a result of accident or disease. Usually these exercises are efficient in regaining motor control, provided the training is intense and the patient is guided in the therapy. Another example of a subject is an athlete.

A rehabilitation system is disclosed in application CN 200410056143.0 (published as CN 1734459 A). In the disclosure of the rehabilitation system, during the exercise, the posture of a patient is captured by two cameras. The parameters, such as the range of movement, physical activity level, etc., acquired by the cameras and/or other sensors, are used to evaluate an actual performance of the patient during one exercise. A performance goal of the exercise, such as target level of the exercise, is predefined by a rehabilitation specialist. The specialist may make a more accurate diagnosis and/or set up a more suitable rehabilitation program for the patient, based on a comparison between the actual performance and the target level.

The rehabilitation program, e.g. target level of an exercise, cannot be adjusted until the patient visits the specialist. The duration may be too long to ensure compliance of the patient. The patient may become de-motivated to do the exercise especially in an unsupervised home rehabilitation program.

WO 2007/140490 A1 discloses a training arrangement with at least one first exercising apparatus and one second exercising apparatus, wherein the first exercising apparatus is connected to a first computer for controlling and/or recording a training programme, and wherein the second exercising apparatus is connected to a second computer for controlling and/or recording a training programme, and wherein the first exercising apparatus is designed differently from the second exercising apparatus. The first computer and the second computer are designed for the exchange of data, in particular control, training and/or personal data, and are preferably connected by a data line.

WO 2009/127788 A1 discloses a method for creating an exercise history for an exerciser to create an exercise program substantially in real-time, wherein personal exercise information is gathered to create the exercise history during an exercise event in an exercise environment having one or more exercise units. The document provides a method and device for creating an exercise program, and a method and device for controlling an exercise event.

**Summary of the Invention**

The invention relates to systems of providing a training program as specified in appended independent claims 1 and 6. Preferred embodiments of the invention are disclosed in the dependent claims.

These and other characteristics, features and advantages of the present invention will become apparent from the following detailed description, taken in conjunction with the accompanying drawings, which illustrate, by way of example, the principles of the invention. The reference numerals given below refer to the attached drawings.

**Brief Description of the Drawings**

The invention is explained in further detail, by way of example and with reference to the accompanying drawings, wherein:

Figure 1 shows a system of providing a training program to a subject according to an embodiment of the invention;
Figure 2 is an illustration of a training program including a series of exercises according to an embodiment of the invention;
Figure 3 illustrates a flowchart for a process of providing a training program to a subject according to an embodiment of the invention;
Figure 4 shows a system of providing a training program to a subject according to another embodiment of the invention; and
Figure 5 illustrates a flowchart for a process of providing a training program to a subject according to another embodiment of the invention.

Throughout the above drawings, like reference numerals will be understood to refer to like, similar or corresponding features or functions.

## Detailed Description

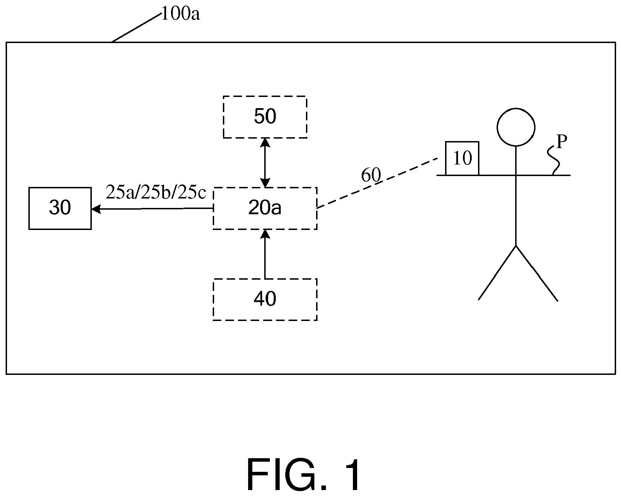

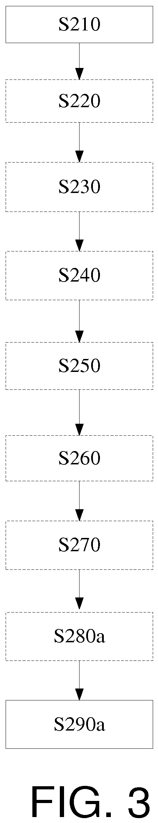

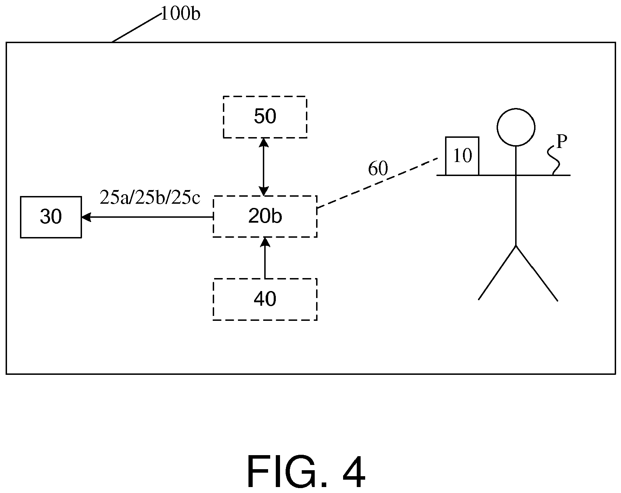

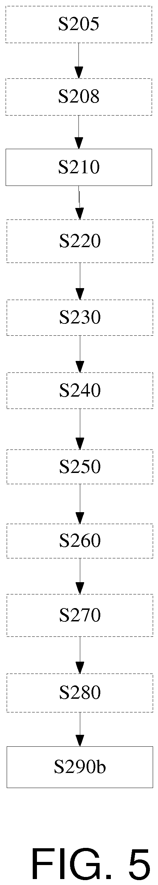

The present invention will be described with respect to particular embodiments and with reference to certain drawings, but the invention is not limited thereto but only by the claims. The drawings described are only schematic and are non-limiting. In the drawings, the size of some of the elements may be exaggerated and not drawn to scale for illustrative purposes. In the block diagrams of Fig 1 and Fig 4, the dashed lines indicate that the element in question may be removed from the system in various embodiments; in the flowcharts of Fig 3 and Fig 5, the dashed lines indicate that the step in question may be removed from the process in various embodiments. Where an indefinite or definite article is used when referring to a singular noun, e.g. "a", "an", "the", this includes a plural of that noun unless something else is specifically stated.

Figure 1 shows a system 100a of providing a training program to a person P according to an embodiment of the invention. The system 100a comprises a sensing device 10, an analyzer 20a and a controller 30. The sensing device 10 may include a plurality of sensors to detect movement of a person P. The sensors can be e.g. inertial sensors, marker-based or markerless camera systems for motion acquisition. During the time that an exercise is performed by the person P, the sensing device 10 is used to monitor the exercise and capture parameters of the movements of the person P. The parameters are supplied to the analyzer 20a via wired or wireless connection 60 between the sensing device 10 and the analyzer 20a.

In an embodiment, the parameters may be represented as a set of progress factors associated with an exercise for a specific training, for example, rehabilitation therapy. The values of the progress factors may be associated with the exercise performance of the person P. In a physical activity exercise for upper limbs, the set of progress factors may include range of movement $PF_a$, speed of movement $PF_b$, smoothness $PF_c$, and trunk stability $PF_d$. An actual performance 25a (i.e. performance level) of the exercise performed by the person P is generated by the analyzer 20a based on analysis of the progress factors $PF_a$, $PF_b$, $PF_c$ and $PF_d$.

In another embodiment, the system 100a further comprises a weight supplier 40 which may supply a set of weight factors to the analyzer 20a based on characteristics of an exercise. In a physical activity exercise for stretching upper limbs, for example, the range of movement $PF_a$ and trunk stability $PF_d$ are used to evaluate the actual performance 25b of the exercise performed by the person P. Correspondingly, weight factors $WF_a$ and $WF_d$ are set to non-zero values, and weight factors $WF_b$ and $WF_c$ are set to zero. The analyzer 20a selects $PF_a$ and $PF_d$ in accordance with the non-zero weights $WF_a$ and $WF_d$. The actual performance 25b is thus evaluated from the progress factors $PF_a$ and $PF_d$.

In addition, an exercise may be performed repeatedly by the person P. The number of repetitions of the exercise may be predetermined or determined by a rehabilitation specialist. In another embodiment, the system 100a may further comprise a storage 50. During the time that an exercise is performed repeatedly, values of progress factors for the exercise are stored in the storage 50. In another embodiment, where it is assumed that an exercise is performed repeatedly for ten times, values of progress factors for one or more mid-repetitions of the exercise, five mid-repetitions for example, are stored in the storage 50. When the person P finishes the exercise, each of the progress factors includes a group of values to be stored in the storage 50. The average of one group of values is calculated as an actual value of one progress factor. The actual values are used to evaluate an actual performance 25c of the exercise performed by the person P. That is to say, the actual performance 25c may be obtained on the basis of actual values for each of progress factors $PF_a$, $PF_b$, $PF_c$ and $PF_d$.

The controller 30 is provided with the actual performance (25a, 25b, or 25c). In the controller 30, the actual performance is compared with a target level of the exercise. An instruction is generated by the controller 30 to adjust the target level of an upcoming exercise, based on the compared result, which will be explained in detail later in the text. The controller 30 provides the upcoming exercise with adjusted target values to the person P through a display (not shown).

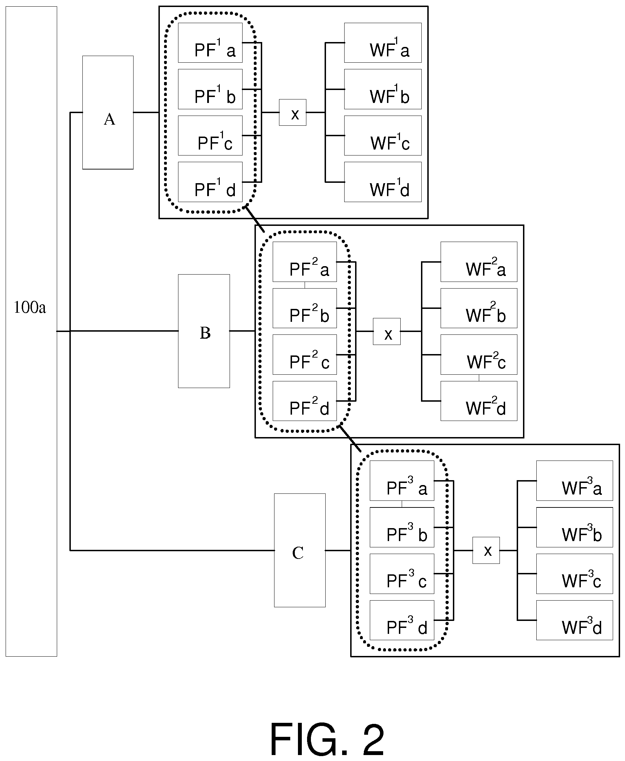

Figure 2 is an illustration of a training program including a series of exercises which can be monitored by the system 100a as shown in Figure 1. In this embodiment, the training program can be used for stroke rehabilitation. The training program includes a plurality of sessions, each session further including a series of exercises, and the series of exercises being intended to be sequentially provided to the subject. As shown in Figure 2, a session of physical activity for upper limbs includes three exercises, i.e. Exercise A for hand movement, Exercise B for wrist rotation and Exercise C for stretching the upper limbs.

An evaluation of the performance level for each of the exercises in the session of physical activity for upper limbs is made on the basis of four progress factors, i.e. range of movement $PF_a$, speed of movement $PF_b$, smoothness $PF_c$, and trunk stability $PF_d$. One or more progress factors are selected by the corresponding weight factors, and in one embodiment, these selected progress factors may be used in the evaluation of the actual performance 25b. Here it is assumed that Exercises A, B and C represent Stages 1, 2 and 3. The upper index *1, 2,* and *3* in progress factor (PF) or weight factor (WF) shown in Figure 2 indicates values of Exercises A, B and C corresponding to stage 1, 2 and 3 respectively.

The following is a table showing a relationship between an evaluation of an actual performance of an exercise, progress factors and weight factors.

Table 1

| Exercise | Progress Factors (PF) Used in Evaluation | Weight Factors (WF) |
|---|---|---|
| Exercise A (hand movement) | $PP^1_a$, $PF^1_c$ | $WF^1_a > 0$, $WF^1_c > 0$<br>$WF^1_b = 0$, $WF^1_d = 0$ |
| Exercise B (wrist rotation) | $PF^2_a$, $PF^2_b$, $PF^2_d$ | $WF^2a > 0$, $WF^2_b > 0$<br>$WF^2_d > 0$, $WF^2_c = 0$ |
| Exercise C (stretching the upper limbs) | $PF3_a$, $PF3_d$ | $WF^3_a > 0$, $WF^3_d > 0$<br>$WF^3_b = 0$, $WF^3_c = 0$ |

Figure 3 illustrates a flowchart for a training program as shown in Figure 2 to the person P. Exercises A, B and C are intended to be sequentially provided to the subject. During the time that Exercise A is provided to the subject, a sensing device 10 monitors Exercise A performed by the person P to acquire progress factors, i.e. range of movement $PF^1_a$, speed of movement $PF^1_b$, smoothness $PF^1_c$, and trunk stability $PF^1_d$ (Step S210).

During the time that the person P performs Exercise A repeatedly, e.g. ten times, values of the progress factors for the five mid-repetitions of Exercise A are stored in the storage 50 (Step S220). Once the person P finishes the Exercise A, average values for each of the progress factors $PF^1_a$, $PF^1_b$, $PF^1_c$ and $PF^1_d$ are calculated as actual values based on the stored values of the progress factors from the five mid-repetitions of Exercise A. The actual values are supplied to the analyzer 20a (Step S230).

Based on characteristics of Exercise A, i.e. hand movement in the session of physical activity for upper limbs, the weight supplier 40 provides a set of weight factors $WF^1_a$, $WF^1_b$, $WF^1_c$ and $WF^1_d$ corresponding to the acquired progress factors $PF^1_a$, $PF^1_b$, $PF^1_c$ and $PF^1_d$ (Step S240). The weight factors are also supplied to the analyzer 20a, wherein the values of $WF^1_a$ and $WF^1_c$ are non-zero, and the values of $WF^1_b$ and $WF^1_d$ are zero (Step S250).

The analyzer 20a, based on the actual values of the progress factors and the weight factors, generates an actual performance (i.e. actual performance level) of Exercise A (Step S260). Since $WF^1_b$ and $WF^1_d$ are zero, $PF^1_a$ and $PF^1_c$ corresponding to non-zero weights $WF^1_a$ and $WF^1_c$, are selected to be used in the evaluation of the actual performance level of Exercise A. Then, the actual performance level is supplied to the controller 30 (Step S270). The controller 30 compares the actual performance level with a target level of Exercise A (Step S280). In this embodiment, the target level for each exercise is determined by a set of target values of the progress factors associated with the exercise. Preferably, initial target values are predefined or predetermined by a specialist.

Based on the comparison result, if the difference between the actual performance level and the target level of Exercise A exceeds a threshold value, the controller 30 generates an instruction to adjust the target level of upcoming Exercise B and/or Exercise C (Step S290a). In particular, if the actual performance level shows the person P performs Exercise A very well and the instruction then indicates to adjust the target level of the next exercise (i.e. Exercise B), target values of progress factors $PF^2_a$ and $PF^2_c$ of Exercise B may be increased based on the selected progress factors $PF^1_a$ and $PF^1_c$ of Exercise A. On the other hand, if the actual performance level shows the person P performs Exercise A poorly and the instruction also indicates to adjust the target level of Exercise B, the target values of the progress factors $PF^2_a$ and $PF^2_c$ of Exercise B may be decreased based on the selected progress factors $PF^1_a$ and $PF^1_c$ of Exercise A. Since a target level of an exercise is determined by target values of progress factors of the exercise, the target level of Exercise B will be adjusted by updating the target values of progress factors thereof.

In an embodiment of the step for adjusting the target level of Exercise B, $PF^2_a$ and $PF^2_c$ of Exercise B are updated by replacing the initial target values of $PF^2_a$ and $PF^2_c$ of Exercise B with the actual values of $PF^1_a$ and $PF^1_c$ of Exercise A respectively. In an alternative embodiment, the initial target values of $PF^2_a$ and $PF^2_c$ of Exercise B are updated by multiplying them by a coefficient which depends upon the actual performance level of Exercise A.

Then, Exercise B, with adjusted target values, is provided to the person P. During the time that the person P performs the next exercise (i.e. Exercise B), a similar process, i.e. Step S210-Step S290a, is performed. The acquired progress factors $PF^2_a$, $PF^2_b$ and $PF^2_d$ are selected to be used in an evaluation of the actual performance level of Exercise B, and whether the target level of the next Exercise C is adjusted depends upon a comparison between the actual performance level of Exercise B and the target level of Exercise B that has been adjusted on the basis of progress factors of Exercise A.

In an embodiment of the step for adjusting the target level of Exercise C, according to the compared result, part of target values of $PF^3_a$, $PF^3_b$ and $PF^3_d$ of Exercise C are updated. As an example, target values of $PF^3_a$ and $PF^3_b$ of Exercise C are replaced by the actual values of $PF^2_a$ and $PF^2_b$ of Exercise B, while the target value of $PF^3_d$ of Exercise C is not updated. Then, Exercise C, with adjusted target values, is provided to the person P.

According to the embodiment described above, the initial target values of $PF^2_a$ and $PF^2_c$ of Exercise B are updated based on the actual values of $PF^1_a$ and $PF^1_c$ of Exercise A. Further, the initial target values of $PF^2_a$ and $PF^3_b$ of Exercise C are updated by the actual values of $PF^2_a$ and $PF^2_b$ of Exercise B. When the person P starts to perform Exercise C, all target values of the progress factors of Exercise C, except $PF^3_d$, are updated. Since at least some of the target values of progress factors of Exercise C are different from the initial target values thereof, the target level of Exercise C is not the initial target level of Exercise C.

Figure 4 shows a system 100b of providing a training program to a person P according to another embodiment of the invention. In comparison with the system 100a illustrated in Figure 1, the system 100b comprises an analyzer 20b. In addition to analyzer 20b, sensing device 10, controller 30, weight supplier 40 and storage 50 in the system 100b, the system (?) can adopt the same or similar devices as the system shown in Figure 1. The detailed description of these same or similar devices is omitted herein.

As illustrated in Figure 4, in addition to an evaluation of the actual exercise performance by the analyzer 20a in Figure 1, the analyzer 20b further comprises a constructor to create a coefficient matrix for updating target values of upcoming exercises. A process performed by the system 100b is illustrated in Figure 5. The process may be described according to an embodiment to provide the training program as shown in Figure 2.

As illustrated in Figure 5, the analyzer 20b obtains training data acquired by the sensing device 10 during the time that a person P performs Exercise A (Step S205). Then, the constructor generates the coefficient matrix by using the training data, based on a linear approximation model (Step S208).

On the supposition that progress factors of each exercise are represented as elements of a matrix, the matrix of stage $m$ and the matrix of stage $m-1$ can be expressed as:

$$\left[PF^m_a, PF^m_b, PF^m_c \ldots PF^m_n\right] = Coff_{n \times n} \left[PF^{m-1}_a, PF^{m-1}_b, PF^{m-1}_c \ldots PF^{m-1}_n\right] \ldots\ldots (1)$$

where the index (e.g. $m$ and $m-1$) indicates the stage of the exercise, $n$ is the number of progress factors associated with a specific exercise, and $Coff_{n \times n}$ represents the coefficient matrix.

In this embodiment, a matrix of Exercise A and a matrix of Exercise B can be shown as:

$$\left[PF^2_a, PF^2_b, PF^2_c, PF^2_d\right] = Coff_{n \times n} \left[PF^1_a, PF^1_b, PF^1_c, PF^1_d\right] \ldots\ldots (2)$$

$Coff_{n \times n}$ is a $4X4$ matrix, thus expression (2) is further represented as:

$$\begin{pmatrix} PF^2_a \\ PF^2_b \\ PF^2_c \\ PF^2_d \end{pmatrix} = \begin{bmatrix} Coff_{11} & Coff_{12} & Coff_{13} & Coff_{14} \\ Coff_{21} & Coff_{22} & Coff_{23} & Coff_{24} \\ Coff_{31} & Coff_{32} & Coff_{33} & Coff_{34} \\ Coff_{41} & Coff_{42} & Coff_{43} & Coff_{44} \end{bmatrix} \begin{pmatrix} PF^1_a \\ PF^1_b \\ PF^1_c \\ PF^1_d \end{pmatrix} \ldots\ldots (3)$$

In accordance with a matrix algorithm, in order to obtain the values of each element in the coefficient matrix, the training data at least includes five groups of progress factors for one exercise, wherein each group of the progress factors can be acquired by sensing device 10 from one repetition of the exercise performed by the person P. In an embodiment, the person P performs the exercise ten times, and the training data is obtained from one or more mid-repetitions of the exercise, for example five mid-repetitions.

Using the training data, the coefficient matrix $Coff_{n \times n}$ is formed based on a linear approximation model, for example minimizing RMSE (root mean square error). After the coefficient matrix $Coff_{n \times n}$ is obtained from the training data, a similar process, i.e. Step S210-Step S280, is performed. The acquired progress factors $PF_a$ and $PF_c$ are selected to be used in an actual performance level evaluation of Exercise A, and whether the target level of Exercise B is adjusted depends upon a comparison between the actual performance level of Exercise A and a target level of Exercise A. Steps S210-S280 may be the same or similar steps as those shown in Figure 3. The detailed description of these steps is omitted herein.

Based on the comparison result, if the difference between the actual performance level of Exercise A and the target level of Exercise A exceeds a threshold value, the controller 30 generates an instruction to adjust the target level of upcoming Exercise B (Step S290b). In Step S290b, target values of the progress factors $PF^2_a$, $PF^2_b$, $PF^2_c$ and $PF^2_d$ of Exercise B are obtained in accordance with Expression (3). As compared with Step S290a in Figure 3, each of the actual values of progress factors $PF^1_a$, $PF^1_b$, $PF^1_c$ and $PF^1_d$ of Exercise A makes contributions to target values of the progress factors of Exercise B. That is to say, the target values of the progress factors of Exercise B are adjusted by a combination of actual values of progress factors $PF^1_a$, $PF^1_b$, $PF^1_c$ and $PF^1_d$ of Exercise A. In other words, even though $PF^1_b$ and $PF^1_d$ of Exercise A are not selected to be used for an evaluation of the actual performance level of Exercise A according to non-zero weights, each one of the progress factors of Exercise A, including $PF^1_b$ and $PF^1_d$, exerts an influence on the target values of the progress factors of Exercise B.

Then, Exercise B, with adjusted target values, is provided to the person P. During the time that the person P performs next Exercise B, a similar process from Step S210- Step S290b is performed. Target values of the progress factors $PF^3_a$, $PF^3_b$, $PF^3_c$ and $PF^1_d$ of Exercise C are updated by multiplying actual values of progress factors $PF^2_a$, $PF^2_b$, $PF^2_c$ and $PF^2_d$ of Exercise B with the coefficient matrix $Coff_{n \times n}$ generated on the basis of training data acquired in Exercise A. So, the target level of Exercise C is adjusted according to the actual values of progress factors of Exercise B.

The system of providing a training program to a subject should not be limited to embodiments mentioned above. It will be apparent to those skilled in the art that the various aspects of the invention claimed may be practiced in other examples that depart from these specific details.

In an alternative embodiment of the system 100a, the analyzer 20a may be removed from the system. Accordingly, the step for performing an actual performance evaluation of Exercise A (Step S260) and the step for supplying the actual performance level (Step 270) are not performed. The comparing step 280 as shown in Fig 3 or Fig 5 may be modified as step 280b accordingly.

After actual values for each of the progress factors $PF_a$, $PF_b$, $PF_c$ and $PF_d$ are supplied to the controller 30 (Step 230) and the weight factors, provided by the weight supplier 40, are also supplied to the controller 30 (step 240), the step 280b may be performed in other embodiments as described below.

In an embodiment, the controller 30 generates an instruction to update target values of Exercise B. The controller 30 selects corresponding actual values of the progress factors of Exercise A according to non-zero weights provided by the weight supplier 40. The target values of Exercise B are then replaced by the corresponding actual values of the progress factors of Exercise A. In another embodiment, the controller 30 compares actual values of the progress factors of Exercise A with target values of Exercise B first. In still another embodiment, actual values of the progress factors of Exercise A provided with non-zero weights are compared with target values of Exercise B. Whether the target values of Exercise B are adjusted depends upon the comparison result. For example, $PF^1_a$ and $PF^1_c$ of Exercise A are compared with $PF^2_a$ and $PF^2_c$, respectively, of Exercise B. Based on the comparison results, if the difference between $PF^1_a$ of Exercise A and $PF^2_a$ of Exercise B exceeds a threshold value, while the difference between $PF^1_c$ of Exercise A and $PF^2_c$ of Exercise B does not exceed a threshold value, only $PF^2_a$ of Exercise B is replaced by $PF^1_a$ of Exercise A. That is to say, each of the target values of Exercise B may be adjusted by the result of the comparison between the actual values of the progress factors of Exercise A and the corresponding target values of Exercise B.

In addition, the order of the steps should not be limited to the procedure shown in Figure 3 and Figure 5. In another embodiment of the training program to a subject, the step for generating weight factors (i.e. Step 240) and supplying the weight factors to the analyzer (i.e. Step 250) for example, may be performed before the step for supplying the actual values of progress factors to the analyzer (i.e. Step 230).

Moreover, the coefficient matrix described in system 100b may be created based on training data associated with each one of the exercises. That is to say, a new coefficient matrix may be generated using training data acquired at the start of an upcoming exercise. The new coefficient matrix is used to adjust target values of the upcoming exercise. In an alternative embodiment, a coefficient matrix may be shared by exercises included in one session. The new coefficient matrix is generated using training data acquired at the start of the exercise in the next session performed by a subject.

Additionally, as described in the embodiment mentioned above, the coefficient matrix is formed based on a linear approximation model. However, other mathematical models in a neural network for example may be applied as well to generate the coefficient matrix based on the training data.

Further, the operation of updating target values of upcoming exercises based on progress factors acquired in an exercise may be performed at the end of the exercise or at start of the upcoming exercise alternatively.

Additionally, the analyzer 20a, the controller 30 and the weight supplier 40 are described as separate modules in the embodiments as shown in Figures 1 and 4. However, a person skilled in the art may understand that the functions of these modules can be implemented by a processor and a readable medium on which a software program including a set of instructions is recorded. When the instructions are executed, the processor will be enabled to perform any one of the steps described in the above-mentioned embodiments.

It should be noted that the above described embodiments are given for describing rather than limiting the invention, and it is to be understood that modifications and variations may be resorted to without departing from the scope of the invention as those skilled in the art readily understand. Such modifications and variations are considered to be within the scope of the invention as defined by the appended claims. The protective scope of the invention is defined by the accompanying claims. In addition, any of the reference numerals in the claims should not be interpreted as a limitation to the claims.

**Claims**

1. A system of providing, to a subject, a training program including at least a first exercise and a second exercise to be provided to the subject after said first exercise, the system comprising:

   - a sensing device (10) for acquiring actual values of a first set of progress factors associated with the first exercise, wherein said actual values are obtained from one or more repetitions of said first exercise;
   - an analyzer (20a, 20b) for evaluating an actual performance level of the first exercise performed by the subject on the basis of at least a portion of the acquired actual values of the first set of progress factors; and
   - a controller unit (30) for

     comparing said actual performance level and a target performance level of the subject performing the first exercise to determine if a difference between said actual performance level and said target performance level of the subject performing the first exercise exceeds a first threshold value, and

     adjusting, based on the result of this comparison, a target value of a progress factor of a second set of progress factors associated with the second exercise, wherein the progress factor, of the second set of progress factors, for which the difference between said actual performance level and said target performance level of the subject performing the first exercise exceeded the first threshold value is adjusted for the second exercise.

2. The system of claim 1, wherein the first and second set of progress factors include range of movement, speed of movement, smoothness and trunk stability.

3. The system of any one of claims 1 to 2, further comprising:

   - a weight supplier (40) for providing a weight factor corresponding to each progress factor of the first set of progress factors, said provided weight factors being associated with characteristics of said first exercise;
   wherein said analyzer (20a, 20b) is configured to further evaluate the actual performance level of the subject performing the first exercise based on the at least a portion of the acquired actual values and said provided weight factors.

4. The system of claim 1, wherein the sensing device (10) comprises a plurality of sensors for detecting movement of the subject to evaluate the actual values of the first set of progress factors.

5. The system of claim 1, wherein the training program further comprises a third exercise, and the control unit is further configured to adjust at least a portion of the target values of a third set of progress factors associated with the third exercise to be provided to the subject subsequent to the first and second exercise based on an actual performance level of the subject performing the second exercise.

6. A system of providing, to a subject, a training program including at least a first exercise and a second exercise to be provided to the subject subsequent to the first exercise, the system comprising:

   - a sensing device (10) for acquiring actual values of a first set of progress factors associated with the first exercise being performed one or more times by the subject; and
   - a controller unit (30) for

     comparing said actual values of the first set of progress factors with one or more target values of a second set of progress factors associated with the second exercise to determine if one or more differences between one or more actual values of the first set of progress factors and one or more target values of the second set of progress exceed one or more respective second threshold values, and

     adjusting, based on the result of this comparison, a target value of a progress factor of a second set of progress factors assocaited with the second exercise, wherein the progress factor, of the second set of progress factors, for which the difference between one or more actual values of the first set of progress factors and one or more target values of the second set of progress exceeds one or more respective second threshold values is adjusted for the second exercise.

7. The system of claim 6, wherein the first and second set of progress factors include range of movement, speed of movement, smoothness and trunk stability.

**Patentansprüche**

1. System zur Bereitstellung eines Schulungsprogramms für ein Subjekt, das mindestens eine erste Übung und eine zweite Übung umfasst, die dem Subjekt nach dieser ersten Übung zur Verfügung gestellt werden soll, wobei das System umfasst:

   - eine Erfassungsvorrichtung (10) zum Erfassen von tatsächlichen Werten eines ersten Satzes von Fortschrittsfaktoren, die mit der ersten Übung zugeordnet sind, wobei die tatsächlichen Werte aus einer oder mehreren Wiederholungen der ersten Übung erhalten werden;
   - einen Analysator (20a, 20b) zur Bewertung eines tatsächlichen Leistungsniveaus der ersten vom Subjekt durchgeführten Übung, auf der Grundlage mindestens eines Teils der erfassten tatsächlichen Werte des ersten Satzes von Fortschrittsfaktoren; und
   - eine Steuereinheit (30) für

   Vergleichen des tatsächlichen Leistungsniveaus und eines Zielleistungsniveaus des Subjekts, das die erste Übung durchführt, um festzustellen, ob ein Unterschied zwischen dem tatsächlichen Leistungsniveau und dem Zielleistungsniveau des Subjekts, das die erste Übung durchführt, einen ersten Schwellenwert überschreitet, und

   basierend auf dem Ergebnis dieses Vergleichs, Anpassen eines Zielwerts eines Fortschrittsfaktors eines zweiten Satzes von Fortschrittsfaktoren, der mit der zweiten Übung zugeordnet ist, wobei der Fortschrittsfaktor des zweiten Satzes von Fortschrittsfaktoren, für den die Differenz zwischen dem tatsächlichen Leistungsniveau und dem Zielleistungsniveau des Subjekt, das die erste Übung durchführt, den ersten Schwellenwert überschreitet, für die zweite Übung angepasst ist.

2. System nach Anspruch 1, wobei der erste und zweite Satz von Fortschrittsfaktoren den Bewegungsbereich, die Bewegungsgeschwindigkeit, die Laufruhe und die Rumpfstabilität umfassen.

3. System nach einem der Ansprüche 1 bis 2, ferner umfassend:

   - einen Gewichtslieferant (40) zum Bereitstellen eines Gewichtsfaktors, der jedem Fortschrittsfaktor des ersten Satzes von Fortschrittsfaktoren entspricht, wobei die angegebenen Gewichtsfaktoren mit den Merkmalen der ersten Übung zugeordnet sind; wobei der Analysator (20a, 20b) konfiguriert ist, um das tatsächliche Leistungsniveau des Subjekts, das die erste Übung ausführt, basierend auf dem mindestens einen Teil der erfassten tatsächlichen Werte und den angegebenen Gewichtsfaktoren, weiter zu bewerten.

4. System nach Anspruch 1, wobei die Erfassungsvorrichtung (10) mehrere Sensoren zum Erfassen einer Bewegung des Subjekts umfasst, um die tatsächlichen Werte des ersten Satzes von Fortschrittsfaktoren zu bewerten.

5. System nach Anspruch 1, wobei das Schulungsprogramm ferner eine dritte Übung umfasst, und die Steuereinheit ferner konfiguriert ist, um mindestens einen Teil der Zielwerte eines dritten Satzes von Fortschrittsfaktoren anzupassen, die mit der dritten Übung zugeordnet sind, die dem Subjekt nach der ersten und zweiten Übung zur Verfügung gestellt werden sollen, basierend auf einem tatsächlichen Leistungsniveau des Subjekts, der die zweite Übung durchführt.

6. System zur Bereitstellung eines Schulungsprogramms für ein Subjekt, das mindestens eine erste Übung und eine zweite Übung umfasst, die dem Subjekt nach der ersten Übung zur Verfügung gestellt werden sollen, wobei das System umfasst:

   - eine Erfassungsvorrichtung (10) zum Erfassen von tatsächlichen Werten eines ersten Satzes von Fortschrittsfaktoren, die mit der ersten Übung zugeordnet sind, die ein oder mehrere Male von dem Subjekt durchgeführt wird; und
   - eine Steuereinheit (30) zum

   Vergleichen der tatsächlichen Werte des ersten Satzes von Fortschrittsfaktoren mit einem oder mehreren Zielwerten eines zweiten Satzes von Fortschrittsfaktoren, die mit der zweiten Übung zugeordnet sind, um festzustellen, ob ein oder mehrere Unterschiede zwischen einem oder mehreren tatsächlichen Werten des ersten Satzes von Fortschrittsfaktoren und eines oder mehrerer Zielwerte des zweiten Satzes von Fortschritten einen oder mehrere jeweilige zweite Schwellenwerte überschreiten, und

**EP 2 459 284 B1**

basierend auf dem Ergebnis dieses Vergleichs, Anpassen des Zielwerts eines Fortschrittsfaktors eines zweiten Satzes angepasst von Fortschrittsfaktoren, die mit der zweiten Übung zugeordnet sind, wobei der Fortschrittsfaktor des zweiten Satzes von Fortschrittsfaktoren, für die die Differenz zwischen einem oder mehreren tatsächlichen Werten des ersten Satzes von Fortschrittsfaktoren und einem oder mehreren Zielwerten des zweiten Satzes von Fortschrittsfaktoren einen oder mehrere jeweilige zweite Schwellenwerte überschreitet, für die zweite Übung angepasst ist.

**7.** System nach Anspruch 6, wobei der erste und der zweite Satz von Fortschrittsfaktoren den Bewegungsbereich, die Bewegungsgeschwindigkeit, die Laufruhe und die Rumpfstabilität umfassen.


**Revendications**

**1.** Système pour fournir à un sujet un programme d'entraînement comprenant au moins un premier exercice et un deuxième exercice à fournir au sujet après ledit premier exercice, le système comprenant:

- un dispositif de détection (10) pour acquérir des valeurs réelles d'un premier ensemble de facteurs de progrès associées au premier exercice, où lesdites valeurs réelles sont obtenues à partir d'une ou plusieurs répétitions dudit premier exercice;
- un analyseur (20a, 20b) pour évaluer un niveau de performance réel du premier exercice effectué par le sujet sur la base d'au moins une partie des valeurs réelles acquises du premier ensemble de facteurs de progrès; et
- une unité de commande (30) pour

comparer ledit niveau de performance réel et un niveau de performance cible du sujet effectuant le premier exercice pour déterminer si une différence entre ledit niveau de performance réel et ledit niveau de performance cible du sujet effectuant le premier exercice dépasse une première valeur de seuil, et
ajuster, sur la base du résultat de cette comparaison, une valeur cible d'un facteur de progrès d'un deuxième ensemble de facteurs de progrès associée au deuxième exercice, où le facteur de progrès, du deuxième ensemble de facteurs de progrès, pour lequel la différence entre ledit niveau de performance réel et ledit niveau de performance cible du sujet effectuant le premier exercice a dépassé la première valeur de seuil est ajusté pour le deuxième exercice.

**2.** Système selon la revendication 1, dans lequel les premier et deuxième ensembles de facteurs de progrès comprennent une plage de mouvement, une vitesse de mouvement, une fluidité et une stabilité de tronc.

**3.** Système selon l'une quelconque des revendications 1 à 2, comprenant en outre:

- un fournisseur de poids (40) pour fournir un facteur de poids correspondant à chaque facteur de progrès du premier ensemble de facteurs de progrès, lesdits facteurs de poids fournis étant associés aux caractéristiques de ledit premier exercice;
où ledit analyseur (20a, 20b) est configuré pour évaluer en outre le niveau de performance réel du sujet effectuant le premier exercice sur la base d'au moins une partie des valeurs réelles acquises et desdits facteurs de poids fournis.

**4.** Système selon la revendication 1, dans lequel le dispositif de détection (10) comprend une pluralité de capteurs pour détecter le mouvement du sujet pour évaluer les valeurs réelles du premier ensemble de facteurs de progrès.

**5.** Système selon la revendication 1, dans lequel le programme d'entraînement comprend en outre un troisième exercice, et l'unité de commande est en outre configurée pour ajuster au moins une partie des valeurs cibles d'un troisième ensemble de facteurs de progrès associées au troisième exercice à fournir au sujet après le premier et le deuxième exercice sur la base d'un niveau de performance réel du sujet effectuant le deuxième exercice.

**6.** Système de fourniture, à un sujet, d'un programme d'entraînement comprenant au moins un premier exercice et un deuxième exercice à fournir au sujet après le premier exercice, le système comprenant:

- un dispositif de détection (10) pour acquérir des valeurs réelles d'un premier ensemble de facteurs de progrès associées au premier exercice qui est effectué une ou plusieurs fois par le sujet; et
- une unité de commande (30) pour

9

comparer lesdites valeurs réelles du premier ensemble de facteurs de progrès avec une ou plusieurs valeurs cibles d'un deuxième ensemble de facteurs de progrès associées au deuxième exercice pour déterminer si une ou plusieurs différences entre un ou plusieurs valeurs réelles du premier ensemble de facteurs de progrès et une ou plusieurs valeurs cibles du deuxième ensemble de progrès dépassent une ou plusieurs deuxièmes valeurs de seuil respectives, et
ajuster, sur la base du résultat de cette comparaison, une valeur cible d'un facteur de progrès d'une deuxième ensemble de facteurs de progrès associée au deuxième exercice, où le facteur de progrès, du deuxième ensemble de facteurs de progrès, pour lequel la différence entre une ou plusieurs valeurs réelles du premier ensemble de facteurs de progrès et une ou plusieurs valeurs cibles du deuxième ensemble de progrès dépasse une ou plusieurs deuxièmes valeurs de seuil respectives est ajusté pour le deuxième exercice.

7. Système selon la revendication 6, dans lequel les premier et deuxième ensembles de facteurs de progrès comprennent une plage de mouvement, une vitesse de mouvement, une fluidité et une stabilité de tronc.

FIG. 1

FIG. 2

```
┌─────────────────┐
│      S210       │
└─────────────────┘
         │
         ▼
┌ ─ ─ ─ ─ ─ ─ ─ ─ ┐
      S220
└ ─ ─ ─ ─ ─ ─ ─ ─ ┘
         │
         ▼
┌ ─ ─ ─ ─ ─ ─ ─ ─ ┐
      S230
└ ─ ─ ─ ─ ─ ─ ─ ─ ┘
         │
         ▼
┌ ─ ─ ─ ─ ─ ─ ─ ─ ┐
      S240
└ ─ ─ ─ ─ ─ ─ ─ ─ ┘
         │
         ▼
┌ ─ ─ ─ ─ ─ ─ ─ ─ ┐
      S250
└ ─ ─ ─ ─ ─ ─ ─ ─ ┘
         │
         ▼
┌ ─ ─ ─ ─ ─ ─ ─ ─ ┐
      S260
└ ─ ─ ─ ─ ─ ─ ─ ─ ┘
         │
         ▼
┌ ─ ─ ─ ─ ─ ─ ─ ─ ┐
      S270
└ ─ ─ ─ ─ ─ ─ ─ ─ ┘
         │
         ▼
┌ ─ ─ ─ ─ ─ ─ ─ ─ ┐
      S280a
└ ─ ─ ─ ─ ─ ─ ─ ─ ┘
         │
         ▼
┌─────────────────┐
│     S290a       │
└─────────────────┘
```

# FIG. 3

FIG. 4

```
┌ ─ ─ ─ ─ ─ ─ ─ ┐
      S205
└ ─ ─ ─ ─ ─ ─ ─ ┘
        │
        ▼
┌ ─ ─ ─ ─ ─ ─ ─ ┐
      S208
└ ─ ─ ─ ─ ─ ─ ─ ┘
        │
        ▼
┌───────────────┐
│     S210      │
└───────────────┘
        │
        ▼
┌ ─ ─ ─ ─ ─ ─ ─ ┐
      S220
└ ─ ─ ─ ─ ─ ─ ─ ┘
        │
        ▼
┌ ─ ─ ─ ─ ─ ─ ─ ┐
      S230
└ ─ ─ ─ ─ ─ ─ ─ ┘
        │
        ▼
┌ ─ ─ ─ ─ ─ ─ ─ ┐
      S240
└ ─ ─ ─ ─ ─ ─ ─ ┘
        │
        ▼
┌ ─ ─ ─ ─ ─ ─ ─ ┐
      S250
└ ─ ─ ─ ─ ─ ─ ─ ┘
        │
        ▼
┌ ─ ─ ─ ─ ─ ─ ─ ┐
      S260
└ ─ ─ ─ ─ ─ ─ ─ ┘
        │
        ▼
┌ ─ ─ ─ ─ ─ ─ ─ ┐
      S270
└ ─ ─ ─ ─ ─ ─ ─ ┘
        │
        ▼
┌ ─ ─ ─ ─ ─ ─ ─ ┐
      S280
└ ─ ─ ─ ─ ─ ─ ─ ┘
        │
        ▼
┌───────────────┐
│    S290b      │
└───────────────┘
```

# FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 200410056143
- CN 1734459 A
- WO 2007140490 A1
- WO 2009127788 A1